# EUROPÄISCHE PATENTSCHRIFT

(11) **EP 0 865 259 B1**
(45) Veröffentlichungstag und Bekanntmachung des Hinweises auf die Patenterteilung: **30.05.2001**
(21) Anmeldenummer: 96941647.8
(22) Anmeldetag: 03.12.1996
(51) Int. Cl.: A61B 17/80

(54) **ORTHOPÄDISCHES HALTESYSTEM**
ORTHOPAEDIC FIXING SYSTEM
SYSTEME DE FIXATION ORTHOPEDIQUE

(30) Priorität: 07.12.1995 DE 19545612
(43) Veröffentlichungstag der Anmeldung: 23.09.1998
(73) Patentinhaber: Aesculap AG & Co. KG, 78532 Tuttlingen (DE)
(72) Erfinder: TADDIA, Lino, D-78573 Wurmlingen (DE); ZEPF, Rudolf, D-78604 Rietheim-Weilheim (DE)
(74) Vertreter: Böhme, Ulrich, Dr. Dipl.-Phys.
(86) Internationale Anmeldenummer: EP9605355
(87) Internationale Veröffentlichungsnummer: WO9720513

(56) Entgegenhaltungen:
- WO-A-88/03781
- WO-A-94/26193

## Beschreibung

Die Erfindung betrifft ein orthopädisches Haltesystem mit mindestens einem Implantatteil, das mindestens eine Durchstecköffnung aufweist, und mit stiftförmigen Halteelementen, die in die Durchstecköffnungen eingesetzt werden, wobei die Durchstecköffnungen Erweiterungen aufweisen, die eine elastisch zusammendrückbare kopfförmige Verdickung der Halteelemente aufnehmen, wobei die Erweiterungen einen Außendurchmesser aufweisen, der geringfügig kleiner ist als die nicht elastisch zusammengedrückte kopfförmige Verdickung, und wobei die Erweiterungen Hinterschneidungen aufweisen, in die die kopfförmige Verdickung nach dem Einschieben in die Erweiterung elastisch eingreift, und mit einem Sackloch im Bereich der kopfförmigen Verdickung des Halteelements, in das ein dieses ausfüllender Kern einschiebbar ist.

Derartige Haltesysteme werden zur Verbindung von Knochenfragmenten und zur Fixierung von Skeletteilen verwendet. Es kann sich dabei beispielsweise um Knochenplatten handeln, die mit Knochenschrauben in die Knochensubstanz eingeschraubt und dadurch gegen die Oberfläche des Knochens gedrückt und dort festgelegt werden. Es kann sich auch um Systeme aus mehreren Implantatteilen handeln, die untereinander verbunden werden müssen, beispielsweise verschiedene Knochenplatten oder Knochenplatten einerseits und Endoprothesen andererseits.

In der WO88/03781 ist eine osteosynthetische Vorrichtung beschrieben, bei welcher in eine Knochenplatte eine Knochenschraube elastisch eingerastet werden kann. Die Knochenplatte weist dazu eine Öffnung mit Hinterschneidungen auf, und der Kopf der Knochenschraube ist elastisch zusammendrückbar, so daß er elastisch in diese hinterschnittene Öffnung eingerastet werden kann. Nach dem Einschrauben der Knochenschraube in den Knochen wird die Knochenschraube bei dieser vorbekannten Konstruktion durch Einsetzen eines Verriege lungsstiftes so aufgeweitet, daß die Knochenschraube in der Durchstecköffnung der Knochenplatte im Klemmsitz gehalten wird, sie bildet also nach dem Einsetzen des Verriegelungsstiftes zusammen mit der Knochenplatte eine starre Einheit.

Es ist Aufgabe der Erfindung, ein gattungsgemäßes orthopädisches Haltesystem so auszubilden, daß eine Knochenschraube unverlierbar in der hinterschnittenen Durchstecköffnung der Platte festgelegt werden kann, wobei trotzdem die freie Verdrehbarkeit der Knochenschraube gegenüber der Platte erhalten bleibt.

Diese Aufgabe wird bei einem orthopädischen Haltesystem der eingangs beschriebenen Art erfindungsgemäß dadurch gelöst, daß der Kern so bemessen ist, daß er die kopfförmige Verdickung in seiner in das Sackloch eingeschobenen Endstellung verriegelt ohne sie aufzuweiten.

Dieser einschiebbare Kern, der die Teile der kopfförmigen Verdickung nicht auseinanderdrückt, sondern nur das Sackloch ausfüllt, ohne es aufzuweiten, verhindert, daß die elastisch verbiegbaren Teile der kopfförmigen Verdickung elastisch radial nach innen gebogen werden können, so daß allein das Einschieben dieses Kerns die kopfförmige Verdickung in der Erweiterung der Durchstecköffnung verriegelt, wenn die kopfförmige Verdikkung in die Hinterschneidung der Erweiterung eingreift. Da die kopfförmige Verdickung durch diesen Kern nicht aufgeweitet wird, bleibt die kopfförmige Verdickung frei drehbar in der Erweiterung, die Verriegelung erfolgt allein durch den Formschluß in axialer Richtung.

Diese freie Drehbarkeit der Verdickung in der Erweiterung führt dazu, daß bei Kombinationssystemen die Implantatteile eine gewisse Beweglichkeit gegenüber dem Knochensystem erhalten, obwohl die Halteelemente fest im Knochen oder an anderen Implantatteilen verankert sind. Dadurch wird bei Veränderungen, beispielsweise durch Knochenresorption, verhindert, daß die Implantatteile oder die Halteelemente durch eine ungewollte Krafteinwirkung brechen können.

Grundsätzlich können die Implantatteile die unterschiedlichste Form aufweisen. Beispielsweise kann es sich dabei um Knochenplatten handeln, die mit Hilfe eines Halteelements in Form einer Knochenschraube am Knochen durch Verschraubung gehalten sind und dadurch Knochenfragmente fixieren. Es können auch mehrere Implantatteile durch ein Halteelement unmittelbar miteinander verbunden werden, beispielsweise zwei Knochenplatten oder eine Knochenplatte einerseits und der Schaft einer Endoprothese andererseits.

Weiterhin versteht es sich, daß unter dem Begriff "Knochenplatte" nicht nur rein plattenförmige Bauteile verstanden werden, sondern auch anders geformte Implantate, die zur Festlegung und Stützung des Knochenapparates Verwendung finden. Ebenso umfaßt der Begriff "Knochenschraube" nicht nur im eigentlichen Sinne in Knochensubstanz einschraubbare Haltestifte, sondern stiftförmige Halteelemente mit kopfförmiger Verdickung allgemein, die die Knochenplatten durchsetzen. Diese könnten auch über eine aufgeschraubte Mutter oder andere Zugmittel am Knochenapparat festgelegt werden, sie müssen nicht unbedingt durch eigene Gewindegänge in der Knochensubstanz selbst verankert sein.

Bei einer bevorzugten Ausführungsform der Erfindung kann vorgesehen sein, daß die kopfförmige Verdickung durch mehrere elastisch radial nach innen biegbare Teile des Halteelements gebildet wird.

Dabei ist es vorteilhaft, wenn die elastisch nach innen biegbaren Teile durch radial verlaufende Einschnitte voneinander getrennte, einstückig mit dem Halteelement ausgebildete Abschnitte des Halteelements sind. Es genügt also grundsätzlich bereits, wenn ein Halteelement aus elastischem Material im Bereich der kopfförmigen Erweiterung einen oder mehrere radial verlaufende Einschnitte aufweist, die eine geringfügige Annäherung der Teile des Halteelements ermöglichen, um die geforderte Zusammendrückbarkeit zu realisieren.

Es kann vorgesehen sein, daß das Sackloch sich zum freien Ende des Halteelements hin stufig verengt. Dabei kann sich das Sackloch bis in den Schaft des Halteelements erstrecken, wobei dann der Kern einen entsprechenden Verlängerungsstift trägt, der das Sackloch im wesentlichen vollständig ausfüllt.

Günstig ist es, wenn das Sackloch kreiszylindrisch ausgebildet ist.

Bei einer besonders bevorzugten Ausführungsform ist vorgesehen, daß die Erweiterung eine kugelringförmige Innenfläche und die kopfförmige Verdickung eine komplementäre kugelringförmige Außenfläche aufweisen, deren größter Durchmesser im Innern des Implantatteils im Abstand von dessen Außenfläche angeordnet ist.

Eine solche Ausgestaltung ermöglicht es, das Halteelement in der Erweiterung der Durchstecköffnung über einen bestimmten Winkelbereich zu verschwenken, so daß es schräg zum Implantatteil eingeschraubt werden kann. Trotzdem bleibt das Halteelement in dem Implantatteil gehalten. Dies gilt auch dann, wenn ein Kern in eine Sacklochbohrung eingesetzt ist, der das Halteelement gegen ein Herausziehen aus dem Implantatteil verriegelt. Eine freie Drehbarkeit um die Längsachse des Halteelements und um in der Ebene des Implantatteils liegende Drehachsen bleibt aber dabei voll erhalten.

Bei einer besonders bevorzugten Ausführungsform ist vorgesehen, daß das Halteelement eine Knochenschraube ist.

Bei einer abgewandelten Ausführungsform kann das Halteelement eine Schraube mit einem Maschinengewinde sein. Während sich die Knochenschraube zum Eindrehen in Knochensubstanz eignet, kann ein Halteelement mit einem Maschinengewinde verwendet werden, um zwei Implantatteile miteinander zu verbinden. Es ist dann günstig, wenn ein Implantatteil ein Innengewinde aufweist, in das das Halteelement eingeschraubt werden kann, während das andere Implantatteil in der beschriebenen Weise eine Durchstecköffnung mit einer hinterschnittenen Erweiterung für den Kopf des Halteelements aufweist. Beispielsweise können auf diese Weise zwei Knochenplatten miteinander verbunden werden. Es ist auch möglich, an einem Röhrenknochen den in diesen Knochen eingeführten Schaft einer Endoprothese mit einer außen am Knochen anliegenden Knochenplatte zu verbinden, wobei das Halteelement dann durch die Wand des Knochens hindurchtritt.

Die nachfolgende Beschreibung einer bevorzugten Ausführungsform der Erfindung dient im Zusammenhang mit der Zeichnung der näheren Erläuterung. Es zeigen:

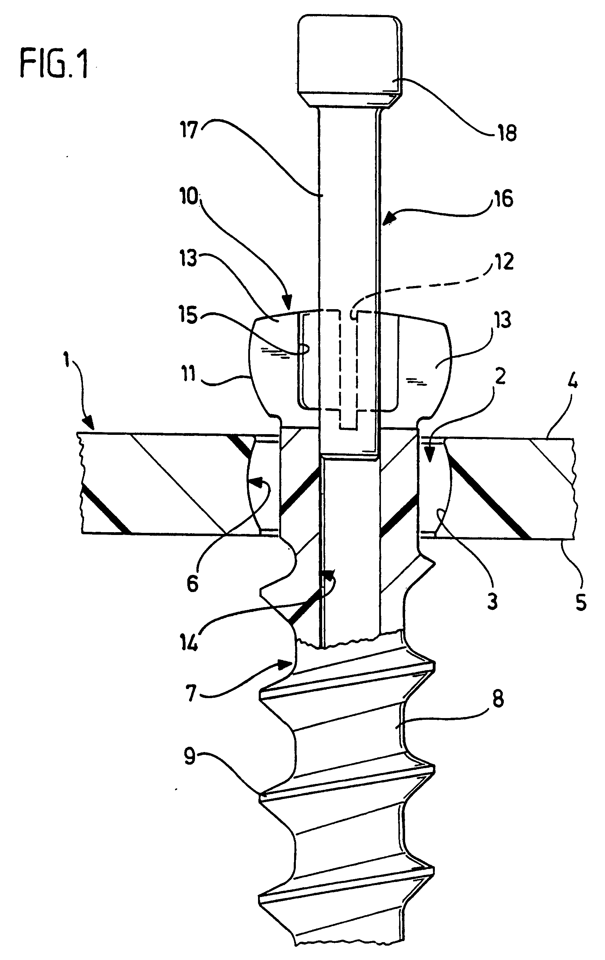

- Figur 1:: eine seitliche Ansicht einer Knochenplatte und einer in einer Durchstecköffnung der Knochenplatte eingesetzten Knochenschraube mit Kern in einer Teilschnittdarstellung vor dem Eindrücken der Knochenschraube in die Durchstecköffnung;

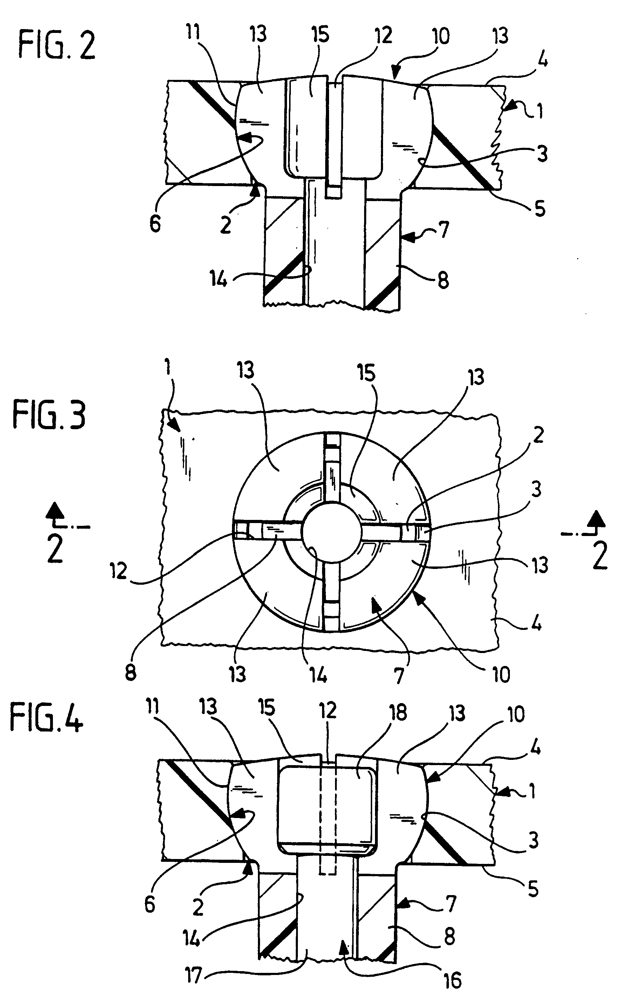

- Figur 2:: eine Ansicht ähnlich Figur 1 der in die Knochenplatte eingedrückten, aber in axialer Richtung nicht verriegelten Knochenschraube der Figur 1 im Bereich der Knochenplatte und der kopfförmigen Verdickung in einem Schnitt längs Linie 2-2 in Figur 3;
- Figur 3:: eine Draufsicht auf die Knochenplatte und die eingesetzte Knochenschraube der Figur 2 und
- Figur 4:: eine Ansicht ähnlich Figur 2 mit eingesetztem Kern zur Verriegelung der Knochenschraube in axialer Richtung.

Das orthopädische Haltesystem wird nachstehend am Beispiel einer Knochenplatte beschrieben, die mit Hilfe von Knochenschrauben an Knochensubstanz festgelegt werden soll. Es versteht sich aber, daß die Erfindung nicht auf den Einsatz an Knochenplatten und die Verwendung von Knochenschrauben beschränkt ist, sondern allgemein auf orthopädische Haltesysteme gerichtet ist, bei denen Implantatteile mit Hilfe von Halteelementen am Knochensystem oder untereinander festzulegen sind.

Das in der Zeichnung dargestellte orthopädische Haltesystem umfaßt eine metallische Knochenplatte 1 mit mehreren Durchstecköffnungen 2, von denen in der Zeichnung nur eine dargestellt ist. Diese Durchstecköffnung hat eine kugelringförmige Innenwand 3, wobei der Bereich des größten Durchmessers der Durchstecköffnung 2 etwa in der Mitte der Knochenplatte 1 angeordnet ist, so daß sich also die Durchstecköffnung 2 zur Oberseite 4 und zur Unterseite 5 hin jeweils verengt. Somit bildet die kugelringförmige Innenwand 3 eine Ausbauchung und damit eine Hinterschneidung 6 aus.

In die Durchstecköffnung 2 ist eine Knochenschraube 7 eingesetzt, die beispielsweise aus einem elastischen Kunststoff bestehen kann, insbesondere aus einem resorbierbaren Kunststoff.

Diese Knochenschraube 7 weist einen Schaft 8 mit einem Außengewinde 9 sowie eine kopfförmige Verdickung 10 auf, die seitlich begrenzt wird durch eine kugelringförmige Umfangsfläche 11, deren Abmessungen im wesentlichen den Abmessungen der ebenfalls kugelringförmigen Innenwand 3 der Durchstecköffnung 2 entsprechen. Die Umfangsfläche 11 ist also komplementär zur Innenwand 3 ausgebildet.

Zwei senkrecht zueinander angeordnete, diametrale Einschnitte 12, die von oben her über die gesamte Höhe der kopfförmigen Verdickung 10 in die Knochenschraube 7 eintauchen, teilen die kopfförmige Verdickung 10 in vier in Umfangsrichtung voneinander getrennte Abschnitte 13 und erzeugen somit eine gewisse Elastizität dieser Abschnitte 13, d.h. die Abschnitte 13 können elastisch radial nach innen gebogen werden.

In die Knochenschraube 7 ist eine zentrale Sacklochbohrung 14 eingearbeitet, die sich bis in den Schaft 8 hineinerstreckt und die im Bereich der kopfförmigen Verdickung 10 einen erweiterten Bereich 15 aufweist. Dieser erweiterte Bereich 15 erleichtert das elastische Zusammendrücken der kopfförmigen Verdickung 10, bei der die Abschnitte 13 elastisch nach innen gebogen werden. Durch dieses Zusammenpressen der kopfförmigen Verdikkung 10 ist es möglich, deren Außendurchmesser zu reduzieren, so daß die kopfförmige Verdickung 10 in die Durchstecköffnung 2 eingeschoben werden kann, obwohl diese an den engeren Übergangsstellen zur Oberseite 4 und zur Unterseite 5 einen Innendurchmesser aufweist, der kleiner ist als der Außendurchmesser der unverformten, nicht zusammengedrückten kopfförmigen Verdickung 10. Beim Einschieben dieser kopfförmigen Verdickung 10 wird diese elastisch zusammengepreßt und schnappt nach dem Eindrücken wieder elastisch auseinander, wobei die bauchige Umfangsfläche 11 in die Hinterschneidung 6 der Durchstecköffnung 2 eingreift. Dadurch wird die Knochenschraube 7 in der Durchstecköffnung 2 in axialer Richtung festgelegt, ohne allerdings die freie Drehbarkeit der Knochenschraube um ihre Längsachse und zusätzlich auch um in der Ebene der Knochenplatte 1 liegende Schwenkachsen zu beeinträchtigen. Damit wird eine drehgelenkartige Verbindung zwischen Knochenschraube 7 und Knochenplatte 1 hergestellt.

Die Knochenschraube 7 kann in geeigneter Weise durch ein Werkzeug verdreht werden, dieses kann beispielsweise in die Einschnitte 12 eingreifen oder in die Sacklochbohrung 14, die zu diesem Zweck einen unrunden Querschnitt haben kann, beispielsweise einen Sechskantquerschnitt.

In die Sacklochbohrung 14 ist ein Kern 16 einsetzbar, der so geformt ist, daß er die Sacklochbohrung 14 im wesentlichen vollständig ausfüllt. Der Kern 16 weist einen länglichen Stift 17 auf, der in den unteren, im Schaft 8 angeordneten Teil der Sacklochbohrung 14 eingreift, und eine zylindrische Verdickung 18, die in den erweiterten Bereich 15 der Sacklochbohrung 14 eintaucht. Dabei sind die Abmessungen so gewählt, daß der Kern 16 in die Sacklochbohrung 14 eintritt, ohne diese aufzuweiten.

Wenn der Kern 16 in dieser Weise in die Knochenschraube 7 eingesetzt ist, wird durch die vollständige Ausfüllung des erweiterten Bereichs 15 der Sacklochbohrung 14 die elastische Verbiegung der Abschnitte 13 radial nach innen behindert, d.h. es ist nicht mehr möglich, den Außendurchmesser der kopfförmigen Verdickung 10 der Knochenschraube 7 zu reduzieren. Wenn die Knochenschraube 7 in die Durchstecköffnung 2 eingesetzt ist, ist es daher nicht mehr möglich, die kopfförmige Verdickung 10 aus der Hinterschneidung 6 zu entfernen, man erhält auf diese Weise also eine Verriegelung der Knochenschraube 7 in der Knochenplatte 1, ohne daß jedoch eine Verklemmung erfolgt. Trotz dieser Verriegelung bleibt die Knochenschraube frei drehbar und frei verschwenkbar in der Durchstecköffnung 2 gehalten, so daß die Drehgelenkeigenschaft der Verbindung zwischen Knochenschraube 7 und Knochenplatte 1 in keiner Weise beeinträchtigt wird.

## Patentansprüche

1. Orthopädisches Haltesystem mit mindestens einem Implantatteil (1), das mindestens eine Durchstecköffnung (2) aufweist, und mit stiftförmigen Halteelementen (7), die in die Durchstecköffnungen (2) eingesetzt werden, wobei die Durchstecköffnungen Erweiterungen aufweisen, die eine elastisch zusammendrückbare kopfförmige Verdickung (10) der Halteelemente (7) aufnehmen, wobei die Erweiterungen einen Außendurchmesser aufweisen, der geringfügig kleiner ist als die nicht elastisch zusammengedrückte kopfförmige Verdickung (10), und wobei die Erweiterungen Hinterschneidungen (6) aufweisen, in die die kopfförmige Verdickung nach dem Einschieben in die Erweiterung elastisch eingreift, und mit einem Sackloch (14) im Bereich der kopfförmigen Verdikkung (10) des Halteelements (7), in das ein dieses ausfüllender Kern (16) einschiebbar ist, dadurch gekennzeichnet, daß der Kern (16) so bemessen ist, daß er die kopfförmige Verdickung (10) in seiner in das Sackloch (14) eingeschobenen Endstellung verriegelt ohne sie aufzuweiten.

2. Haltesystem nach Anspruch 1, dadurch gekennzeichnet, daß die kopfförmige Verdickung (10) durch mehrere elastisch radial nach innen biegbare Teile (13) des Halteelements (7) gebildet wird.

3. Haltesystem nach Anspruch 2, dadurch gekennzeichnet, daß die elastisch nach innen biegbaren Teile (13) durch radial verlaufende Einschnitte (12) voneinander getrennte, einstückig mit dem Halteelement (7) ausgebildete Abschnitte des Halteelements (7) sind.

4. Haltesystem nach einem der voranstehenden Ansprüche, dadurch gekennzeichnet, daß das Sackloch (14) sich zum freien Ende des Halteelements (7) hin stufig verengt.

5. Haltesystem nach einem der voranstehenden Ansprüche, dadurch gekennzeichnet, daß das Sackloch (14) kreiszylindrisch ausgebildet ist.

6. Haltesystem nach einem der voranstehenden Ansprüche, dadurch gekennzeichnet, daß die Erweiterung eine kugelringförmige Innenfläche (3) und die kopfförmige Verdickung (10) eine komplementäre kugelringförmige Außenfläche (11) aufweisen, deren größter Durchmesser im Innern des Implantatteils (1) im Abstand von dessen Außenflächen (4, 5) angeordnet ist.

7. Haltesystem nach einem der voranstehenden Ansprüche, dadurch gekennzeichnet, daß das Halteelement eine Knochenschraube (7) ist.

8. Haltesystem nach einem der Ansprüche 1 bis 7, dadurch gekennzeichnet, daß das Halteelement eine Schraube mit einem Maschinengewinde ist.

9. Haltesystem nach einem der voranstehenden Ansprüche, dadurch gekennzeichnet, daß das Implantatteil eine Knochenplatte (1) ist.

10. Haltesystem nach einem der Ansprüche 1 bis 9, dadurch gekennzeichnet, daß das Halteelement zwei oder mehr Implantatteile miteinander verbindet.

11. Haltesystem nach Anspruch 10, dadurch gekennzeichnet, daß die beiden Implantatteile plattenförmig ausgebildet sind.

12. Haltesystem nach einem der Ansprüche 1 bis 10, dadurch gekennzeichnet, daß ein Implantatteil eine Knochenplatte ist und das andere eine Endoprothese.

## Claims

1. An orthopaedic fixing system having at least one implant part (1) with at least one through opening (2) and having pin-shaped fixing elements (7) which are inserted into the through openings (2), wherein the through openings have widened portions which receive a resiliently compressible head-shaped swelling (10) of the fixing elements (7), wherein the widened portions have an external diameter which is slightly smaller than the head-shaped swelling (10) before resilient compression, and wherein the widened portions have undercuts (6) into which the head-shaped swelling resiliently engages after insertion into the widened portion, and having a blind hole (14) which is located in the region of the head-shaped swelling (10) of the fixing element (7) and into which a core (16) which fills it up can be inserted, characterised in that the core (16) is dimensioned such that in its final position - in which it is inserted into the blind hole (14) - it locks the head-shaped swelling (10) without widening it.

2. A fixing system according to Claim 1, characterised in that the head-shaped swelling (10) is formed by a plurality of fixing-element parts (13) which can be bent radially inwards in a resilient manner.

3. A fixing system according to Claim 2, characterised in that the resiliently inwards bendable parts (13) are portions of the fixing element (7) which are separated from one another by radially-extending slots (12) and which are integral with the fixing element (7).

4. A fixing system according to any one of the preceding Claims, characterised in that the blind hole (14) narrows gradually in the direction of the free end of the fixing element (7).

5. A fixing system according to any one of the preceding Claims, characterised in that the blind hole (14) is circular cylindrical.

6. A fixing system according to any one of the preceding Claims, characterised in that the widened portion has a spherical-annular inner surface (3) and the head-shaped swelling (10) has a complementary spherical-annular outer surface (11), whose largest diameter is arranged within the implant part (1), at a distance from the outer surfaces (4, 5) thereof.

7. A fixing system according to any one of the preceding Claims, characterised in that the fixing element is a bone screw (7).

8. A fixing system according to any one of Claims 1 to 7, characterised in that the fixing element is a screw with a machined thread.

9. A fixing system according to any one of the preceding Claims, characterised in that the implant part is a bone plate (1).

10. A fixing system according to any one of Claims 1 to 9, characterised in that the fixing element connects two or more implant parts to one another.

11. A fixing system according to Claim 10, characterised in that the two implant parts are plate-shaped.

12. A fixing system according to any one of Claims 1 to 10, characterised in that one implant part is a bone plate and the other is an endoprosthesis.

## Revendications

1. Système de fixation orthopédique comportant au moins une partie d'implant (1) qui présente au moins une ouverture traversante (2), et des éléments de fixation (7) en forme de goupilles qui sont mis en place dans les ouvertures traversantes (2), les ouvertures traversantes présentant des évasements qui reçoivent un renflement (10) en forme de tête, élastiquement compressible, des éléments de fixation (7), les évasements présentant un diamètre extérieur qui est légèrement inférieur au renflement (10) en forme de tête lorsqu'il n'est pas élastiquement comprimé, et les évasements présentant des contre-dépouilles (6) dans lesquelles le renflement en forme de tête s'engage élastiquement après avoir été introduit dans l'évasement, et comportant un trou borgne (14) dans la région du renflement (10) en forme de tête de l'élément de fixation (7), trou borgne dans lequel est susceptible d'être enfilé un noyau (16) le remplissant, caractérisé en ce que le noyau (16) a des dimensions telles qu'il verrouille le renflement (10) en forme de tête dans sa position finale enfilée dans le trou borgne (14), sans l'évaser.

2. Système de fixation selon la revendication 1, caractérisé en ce que le renflement (10) en forme de tête est formé par plusieurs parties (13) de l'élément de fixation (7), qui sont susceptibles d'être fléchies élastiquement radialement vers l'intérieur.

3. Système de fixation selon la revendication 2, caractérisé en ce que les parties (13) susceptibles d'être fléchies élastiquement radialement vers l'intérieur sont des tronçons de l'élément de fixation (7) séparés les uns des autres par des encoches (12) s'étendant radialement et réalisés d'un seul tenant avec l'élément de fixation (7).

4. Système de fixation selon l'une quelconque des revendications précédentes, caractérisé en ce que le trou borgne (14) se rétrécit par gradins vers l'extrémité libre de l'élément de fixation (7).

5. Système de fixation selon l'une quelconque des revendications précédentes, caractérisé en ce que le trou borgne (14) est réalisé sous forme de cylindre circulaire.

6. Système de fixation selon l'une quelconque des revendications précédentes, caractérisé en ce que l'évasement présente une surface intérieure (3) en forme de bague sphérique et en ce que le renflement en forme de tête présente une surface extérieure (11) complémentaire en forme de bague sphérique dont le plus grand diamètre est disposé à l'intérieur de la partie d'implant (1), à distance de ses surfaces extérieures (4, 5).

7. Système de fixation selon l'une quelconque des revendications précédentes, caractérisé en ce que l'élément de fixation est une vis à os (7).

8. Système de fixation selon l'une quelconque des revendications 1 à 7, caractérisé en ce que l'élément de fixation est une vis à pas de vis mécanique.

9. Système de fixation selon l'une quelconque des revendications précédentes, caractérisé en ce que la partie d'implant est une plaque à os (1).

10. Système de fixation selon l'une quelconque des revendications 1 à 9, caractérisé en ce que l'élément de fixation relie les uns aux autres deux parties d'implants ou plus.

11. Système de fixation selon la revendication 10, caractérisé en ce que les deux parties d'implants sont réalisées en forme de plaques.

12. Système de fixation selon l'une quelconques des revendications 1 à 10, caractérisé en ce qu'une partie d'implant est une plaque à os et l'autre une endoprothèse.
